# EUROPEAN PATENT APPLICATION

(11) **EP 1 136 468 A2**
(43) Date of publication of application: **26.09.2001**
(21) Application number: 01302508.5
(22) Date of filing: 19.03.2001
(51) Int. Cl.: C07C 203/04, C06B 45/10, C06B 25/10

(54) **Method of synthesizing diglycerol tetranitrate, and solid rocket motor propellant containing the same**

(30) Priority: 23.03.2000 US 191548 P
(71) Applicant: Cordant Technologies, Inc., Salt Lake City, UT 84101-1532 (US)
(72) Inventor: Sanderson, Andrew, North Ogden, Utah 84414 (US); Martins, Laura, Ogden, Utah 84403 (US)
(74) Representative: Stuttard, Garry Philip

(57) **Abstract**

Diglyercol tetranitrate is an energetic nitrate ester plasticizer having no freezing point, making the nitrate ester plasticizer especially suited for use in solid rocket motor propellants that are subjected to low temperature storage and operational environments, which can reach as low as -54°C in temperature. In order to avoid problems associated with fume-off that characterize the conventional synthesis method of making diglycerol tetranitrate, synthesis is performed in a medium including a mixed acid phase and an inert organic phase. The mixed acid phase contains, as ingredients, at least one nitronium ion source and at least one acid having sufficient strength to generate nitronium ions from the nitronium ion source. The nitronium ions in the mixed acid nitrated diglycerol to form diglycerol tetranitrate, which is then received into the organic liquid. The organic liquid, which preferably is a chlorocarbon such as dichloromethane, is insoluble with diglycerol but soluble with diglycerol tetranitrate. The inert organic phase is then treated to neutralize any acid contained in the inert organic phase, and the diglycerol tetranitrate is separated from the inert organic phase.

## Description

### CROSS-REFERENCE TO RELATED APPLICATIONS

The benefit of priority is claimed based on U.S. Provisional Application 60/191,548 filed in the U.S. Patent & Trademark Office on March 23, 2000.

### BACKGROUND OF THE INVENTION

### 1. Field of the Invention

This invention relates to a method of synthesizing diglycerol tetranitrate, which is an excellent plasticizer for energetic materials such as rocket motor propellants, explosives, and pyrotechnics. This invention is also directed to a solid rocket motor propellant comprising diglycerol tetranitrate as the plasticizer.

### 2. Description of the Related Art

Energetic materials used in solid rocket motor propellants, explosives, and pyrotechnics comprise an energetic material that, when ignited, releases sufficient amounts of energy to provide, in the case of a propellant, the interior pressures needed to attain rocket motor flight or, in the case of an explosive, sufficient energy to demolish an intended target. Generally, energetic materials comprise, among other ingredients, a fuel and oxidizing agent immobilized in a polymeric binder. Selection of an appropriate binder can enhance the mechanical properties of the energetic material. Enhanced mechanical properties are important for maintaining the structural integrity of the energetic materials during operation and storage, especially when the energetic materials are subjected to operation and storage conditions characterized by extremely low and high temperatures. Other ingredients are added to the composite solid propellant, as are needed or desired, to provide additional energy performance, improve the mechanical properties of the propellant, and/or simplify processing.

Among the additional ingredients commonly found in energetic materials are plasticizers. In particular, nitrate ester plasticizers have found wide acceptance as energetic plasticizers due to the ability of nitrate ester plasticizers to enhance energetic performance. Nitrate ester plasticizers provide the added benefits of improving rheological properties during processing, preventing crystallization of the binder, and enhancing mechanical properties of the energetic material.

Due to the low temperatures at which energetic materials are sometimes stored, as well as the low temperatures that energetic materials existing as rocket motor propellants experience during high altitude operation, military specifications sometimes require that energetic materials be resistant to prolonged exposure to temperatures as low as -54°C. Inferior low temperature mechanical properties, such as poor tensile strength and low strain capability, can generate mechanical strain in the energetic material at low temperatures and can promote the likelihood of fracture to the energetic material. For instance, in the case of a solid propellant grain, fractures in the propellant grain can, if widespread, significantly increase the propellant grain surface area available for combustion reaction. As a consequence, the chamber pressure created during combustion of a propellant grain can be increased to unanticipated levels, leading in extreme cases to catastrophic failure of the rocket motor in which the propellant grain is loaded upon ignition.

The most commonly used conventional nitrate ester plasticizers reach their freezing points well above the military design specification of -54°C. In particular, nitroglycerin has a freezing point of about -13°C. Butanetrioltrinitrate (BTTN) has a freezing point of -27°C. The freezing point of trimethylolethanetrinitrate (TMETN) is about -15°C. Upon freezing, these common nitrate ester plasticizers tend to crystallize and migrate into agglomerations, disrupting the homogeneity of the energetic material and increasing the risk of fracture to the energetic material.

As reported in Seymour M. Kaye, The Encyclopedia of Explosives and Related Items (U.S. Army Armament Research & Development Command 1983), between 1920 and World War II a currently less used nitrate ester plasticizer, diglycerol tetranitrate, was used in combination with nitroglycerin as a nitrate ester plasticizer for nitrocellulose-based explosives. Unlike nitroglycerin, BTTN, and TMETN, diglycerol tetranitrate does not have a freezing point, much less a freezing point below -54°C. However, to the knowledge of the inventors, use of diglycerol tetranitrate has ceased and publications discussing diglycerol tetranitrate have been few since approximately the end of World War II. The cessation of activity relating to diglycerol tetranitrate is believed by the inventors to be attributable to drawbacks associated with the conventional synthesis method for making diglycerol tetranitrate. Conventional synthesis calls for the nitration of diglycerol in a mixed acid comprising nitric acid to make diglycerol tetranitrate, followed by quenching of the mixed acid in water to recovery the diglycerol tetranitrate. However, a significant portion of the diglycerol tetranitrate formed in the mixed acid is not recovered by quenching the mixed acid. The difficulty in recovering diglycerol tetranitrate by quenching of a mixed acid is responsible not only for relatively low yields of about 80 molar percent, as reported in the art, but also for fume-off problems. Residual nitric acid remaining in the spent mixed acid reacts with unrecovered diglycerol tetranitrate in an exothermic reaction that is autocatalytic. This exothermic reaction generates large amounts of nitrogen oxide and water in a process known as a fume-off. Due to the autocatalytic nature of this reaction and the formation of large amount of nitrogen oxide, if left uncontrolled the fume-off can lead to violent explosion and other problems. Accordingly, great care in the handling and disposal of the waste acid is required to avoid unintentional explosion. Another problem of the conventional synthesis method stems from the solubility of the diglycerol tetranitrate in the mixed acid. During quenching an emulsion tends to form in the spent mixed acid. The emulsion is difficult and slow to separate from the spent mixed acid, thus increasing process time and the likelihood for fume-off.

It would, therefore, be a significant improvement in the art to provide a method of synthesizing diglycerol tetranitrate in which the risk of fume-off is significantly reduced and which provides much higher, almost quantitative yields.

### SUMMARY OF THE INVENTION

An object of this invention is to fulfill a long-felt need in the art by providing a method of synthesizing diglycerol tetranitrate that attains the above-discussed improvement.

In accordance with the principles of this invention, the above and other objects are attained by nitrating diglycerol in a nitrating (or reaction) medium comprising a mixed acid phase and an inert organic phase. The mixed acid phase comprises, as ingredients, at least one nitronium ion source capable of nitrating each of the four hydroxyl groups of diglycerol and at least one acid having sufficient strength to generate nitronium ions from the nitronium ion source. The inert organic phase comprises at least one organic liquid that is immiscible with the mixed acid phase, so that the inert organic phase and mixed acid phase are stratified. The organic liquid should neither dissolve nor nitrate the diglycerol. In practice, the inert organic phase provides a liquid medium for receiving from the mixed acid phase the diglycerol tetranitrate generated by nitration of the diglycerol.

There are several advantages that are derived from practicing the inventive process. For example, the diglycerol tetranitrate has greater solubility in the inert organic phase than the mixed acid phase, and upon synthesis migrates from the mixed acid phase to the inert organic phase. As a consequence, heat and diglycerol tetranitrate generated during the exothermic nitration reaction migrate from the mixed acid phase and into the inert organic phase, thereby reducing the likelihood of fume-off and degradation of diglycerol tetranitrate in the mixed acid phase. Additionally, the migration of the diglycerol tetranitrate into the inert organic phase simplifies and improves the efficiency of diglycerol tetranitrate recovery, thereby providing improved yields compared to the conventional synthesis route. For example, diglycerol tetranitrate yields are routinely on the order of 95% molar or greater according to the inventive method, and often approach or attain quantitative maximum yields. The spent mixed acid phase can then be neutralized and disposed of with lesser concerns over fume off and other safety issues.

Synthesis by the method of this invention is particularly useful for making diglycerol tetranitrate for solid rocket motor propellants, especially tactical propellants designed for enduring storage and operating temperatures as low as -54°C. Additionally, propellants comprising diglycerol tetranitrate have significantly better safety handling properties compared to other energetic plasticizers, such as BTTN. In this regard, diglycerol tetranitrate exhibits a surprisingly low sensitivity to ignition and detonation from accidental impact and shock stimuli. Diglycerol tetranitrate is also characterized by high viscosity and low vapor pressures, improving the handleability and processability of diglycerol tetranitrate in the production of solid rocket motor propellants.

Other objects, aspects, and advantages of this invention will become more apparent to those skilled in the art upon reading the specification and appended claims which, when taken in conjunction with the accompany drawing, explain the principles of this invention.

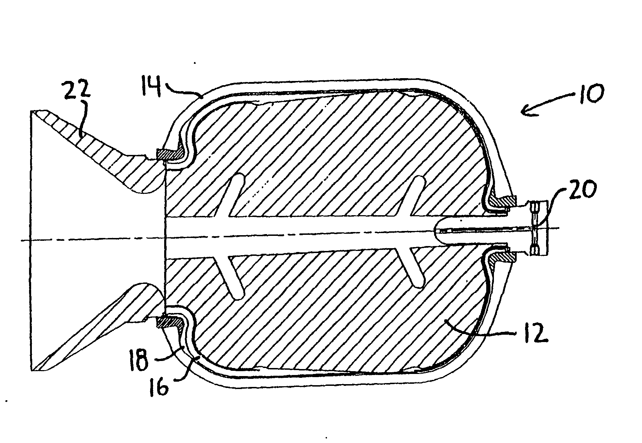

### BRIEF DESCRIPTION OF THE DRAWING

The accompanying drawing serves to elucidate the principles of this invention by illustrating a rocket motor assembly in which a solid propellant comprising diglycerol tetranitrate made in accordance with the method of this invention may be loaded.

### DETAILED DESCRIPTION OF THE INVENTION

Diglycerol is a tetraol ether usually found as a mixture of three isomers: 3,3'-oxy-di(1,2-propanediol), 2,2'-oxy-di(1,3-propanediol), and 2-(hydroxymethyl) -3-oxyhexane-1,5,6-triol. As referred to herein, diglycerol includes one or a combination of more than one of these isomers.

Among nitronium ion sources that can be used in accordance with the present invention to nitrate the diglycerol are nitric acid and nitronium salts, such as nitronium nitrate and nitronium tetrafluoroborate. The generation of nitronium ions from the nitronium ion source is performed with a strong acid. Although sulfuric acid is preferred, other strong acids and anhydrides capable of generating nitronium ions from the nitronium ion source, while being substantially inert with the nitronium ions, may be used as the strong acid in addition to or as an alternative for sulfuric acid. Other acids that can be used to generate nitronium ions from the nitronium ion source include methane sulfonic acid (CH₃SO₂OH), acetic anhydride, acetic acid, and phosphoric acid, as well as combinations thereof.

The molar ratio of nitronium ion source (e.g., nitric acid) to diglycerol is at least a stoichiometric amount of 4:1 (four nitronium ions for the four hydroxyl groups of each diglycerol), but preferably is not greater than about 10:1, more preferably 8:1, for economic reasons.

For practical reasons relating to availability and handleability, preferably nitric acid is used in combination with sulfuric acid to form a mixed acid phase. Various grades of nitric acid and sulfuric acid can be used to make the mixed acid phase, the proportions of mixed acid (nitronium ion source and strong acid) to water in the mixed acid phase can be from about 100:0 to about 80:20 by weight.

Prior to the addition of diglycerol to the mixed acid phase, the mixed acid phase is combined with at least one organic liquid, which is immiscible with the mixed acid and stratifies the mixed acid phase to form a separate inert organic phase. The inert organic phase preferably comprises dichloromethane (also known as methylene chloride), although other chlorocarbons such as chloroform and dichloroethane may be used alone or in combination with dichloromethane to provide the inert organic phase. Further, the chlorocarbons of the inert organic phase should be inert and non-solvents with respect to the diglycerol reagent, yet should be capable of dissolving the diglycerol tetranitrate into solution without degrading (or solvolyzing) the diglycerol tetranitrate. One of the advantages associated with the presence of the inert organic phase is that upon synthesis, the diglycerol tetranitrate product is removed from the mixed acid phase as the product is received into the inert organic phase, thus eliminating the risks and hazards of fume-off that would otherwise be associated with retention of the diglycerol tetranitrate in the mixed acid phase.

A sufficient volume of inert organic phase should be present to permit all of the diglycerol tetranitrate product to be received into the inert organic phase. On the other hand, there is no upper limit on the amount of inert organic phase, except as dictated by economic inefficiencies and waste management. The weight ratio of inert organic phase to diglycerol tetranitrate is preferably at least 1:1.

During the addition of the diglycerol to the nitrating medium, the temperature of the nitrating medium is preferably maintained in a range of from about 5°C to about 20°C, more preferably 10°C to 15°C. This may be accomplished, for example, by conducting the nitration reaction in a jacketed reactor.

The inert organic phase containing the diglycerol tetranitrate can be separated from the mixed acid phase by liquid/liquid separation techniques known in the art, including phase inversion, in which a sufficient amount of water is added to the reaction medium to quench the mixed acid phase and cause the inert organic phase to become denser than the mixed acid phase. Separation funnels can be used to separate the inverted phases.

Any residual acid in the separated inert organic phase can then be neutralized by addition of one or more suitable neutralization agents, typically in the form of a weak base or weak bases. Representative neutralization agents include: carbonates, such as sodium carbonate and potassium carbonate, and calcium carbonate; and bicarbonates, such as sodium bicarbonate, and potassium bicarbonate.

The glycerol tetranitrate can then be separated from the organic liquid (e.g., methylene chloride), for example, by evaporation. In the case of the use of methylene chloride, evaporation can be performed under reduced pressures at temperatures of about 30°C.

The diglycerol tetranitrate is especially useful as a plasticizer for solid rocket motor propellants, including elastomer-based composite propellants, modified composite propellants, crosslinked double-base propellants, and other plasticized propellants.

Representative nitrate ester plasticizers that optionally can be used in combination with the diglycerol tetranitrate to further plasticize the energetic composition include, by way of example, nitroglycerine, trimethylolethanetrinitrate, triethyleneglycoldinitrate, diethyleneglycoldinitrate, ethyleneglycol dinitrate, butanetrioltrinitrate, alkyl NENA's, or combinations thereof. The propellant can also include one or more inert plasticizers, such as triacetin (glycerol triacetate), dioctyladipate, isodecylperlargonate, dioctylphthalate, dioctylmaleate, dibutylphthalate, di-n-propyl adipate, diethylphthalate, dipropylphthalate, CITROFLEX® , diethyl suberate, diethyl sebacate, diethyl pimelate, or combinations thereof.

Solid rocket motor propellants also commonly include inorganic oxidizers and metal fuels. Representative inorganic oxidizers include, by way of example, ammonium perchlorate, ammonium nitrate, hydroxylammonium nitrate, ammonium dinitramide, potassium dinitramide, potassium perchlorate, or combinations thereof. Representative fuels include metals, such as aluminum, magnesium, boron, titanium, silicon, and alloys and/or mixtures thereof. The fuel and oxidizer may be present as powder, or in particulate or other forms. Other ingredients known in the art that optionally can be included in the solid propellant in various combinations include the following: bonding agents such as TEPANOL; energetic fillers such as nitramines; thermal stabilizers such as N-methyl-p-nitroaniline; ballistic modifiers such as titanium dioxide, lead compounds, and bismuth compounds; reinforcing fibers; and pressure oscillation stabilizers, such as zirconium carbide and alumina. Methods of making and casting solid rocket motor propellants, as well as acceptable combinations and concentrations of ingredients, are within the purview of those skilled in the art of rocket motor science.

An example of a rocket motor assembly comprising a solid rocket motor propellant containing diglycerol tetranitrate is shown in the Figure, in which the rocket motor assembly is generally designated by reference numeral 10. The assembly 10 includes a solid propellant grain 12 loaded within the interior surface of the rocket motor case 14. Typically, insulation 16 and a liner 18 are interposed between the case 14 and the solid propellant grain 12. The insulation 16 and the liner 18 serve to protect the case from the extreme conditions produced during combustion of the solid propellant grain 12. Methods for loading a rocket motor case 14 with the insulation 16, the liner 18, and the solid propellant grain 12 are known to those skilled in the art, and can be readily adapted without undue experimentation to incorporate the propellant of this invention. Liner compositions and methods for applying liners into a rocket motor case are also well known in the art. Also shown in the Figure is an igniter 20 attached to the forward end of the case 14 for igniting the solid propellant grain 12 and a nozzle assembly 22 attached at the aft end of the case 14 for expelling at high velocities combustion products generated during burning of the solid propellant grain 12.

The following examples are offered to further illustrate the synthesis methods of the present invention. The examples are intended to be exemplary and should not be viewed as exhaustive of the scope of the invention.

### EXAMPLES

### Example 1

Sulfuric acid (96%, 20 ml) was added to nitric acid (90%, 20 ml), then cooled to below 38°C before adding methylene chloride (100 grams). This mixture was cooled to 0°C in an ice bath and 8 grams of diglycerol were added dropwise over 0.5 hour. At this temperature of 0°C, the diglycerol tended to coagulate despite rapid agitation of the reaction mixture. After another 0.5 hour the coagulated diglycerol had disappeared and the reaction mixture was poured onto 40 ml of crushed ice, which dissolved to form a dilute acid. The organic phase was separated from the dilute acid with a separation funnel and was washed with 50 ml of saturated sodium bicarbonate solution. The organic phase was then dried over 5 grams of magnesium sulfate, filtered, and the volatiles were removed at 30°C under reduced pressure to give diglycerol tetranitrate as a pale yellow oil in 95% yield.

### Example 2

Sulfuric acid (96%, 30 ml) was added to nitric acid (90%, 30 ml), then cooled to below 38°C before adding 100 ml of methylene chloride. This mixture was further cooled to 10°C and 12 g of diglycerol were added by pouring in a steady thin stream over 15 minutes. The rate of addition was such that with a cooling bath at 0°C the reaction temperature stayed between 10°C and 12°C. Under these conditions the diglycerol did not coagulate. After a further 15 minutes the reaction mixture was poured onto 200 ml of crushed ice to dissolve the ice and form a dilute acid. The organic phase was separated from the dilute acid with a separation funnel and was washed with 50 ml of saturated sodium bicarbonate solution. The organic phase was then dried over 5 grams of magnesium sulfate, filtered, and the volatiles were removed at 30°C under reduced pressure to give diglycerol tetranitrate as a pale yellow oil in 96% yield.

### Example 3

Sulfuric acid (96%, 120 ml) was added to nitric acid (90%, 120 ml), then cooled to below 38°C before adding 400 ml of methylene chloride. This mixture was cooled to 5°C and 48.4 grams of diglycerol were added by pouring in a steady thin stream over 15 minutes. The rate of addition was such that with a cooling bath at 0°C the reaction temperature stayed at 5°C. Under these conditions the diglycerol did not coagulate, but the diglycerol did tend to form a film on the reactor sides and thermometer before it reacted and dissolved. After a further 15 minutes the reaction mixture was poured onto 1 liter of crushed ice to form a dilute acid. The organic phase was separated from the dilute acid with a separation funnel and was washed twice with 500 ml of saturated sodium bicarbonate solution. The organic phase was then dried over 5 grams of magnesium sulfate, filtered, and the volatiles were removed at 30°C under reduced pressure to give diglycerol tetranitrate as a pale yellow oil in 96% yield.

### Example 4

Sulfuric acid (96%, 1200 ml) was added to nitric acid (90%, 1200 ml), then cooled to below 38°C before adding 3000 ml of methylene chloride. This mixture was cooled to 10°C and 460 g of diglycerol were added by pouring in a steady thin stream over 65 minutes. The rate of addition was such that with a cooling bath at 0°C the reaction temperature stayed between 10°C and 15°C. Under these conditions the diglycerol did not coagulate. After a further 30 minutes the organic phase was separated from the acid phase without dilution. The organic phase was washed twice with 500 ml of saturated sodium bicarbonate solution. The organic phase was then dried over 5 grams of magnesium sulfate, filtered, and the volatiles were removed at 30°C under reduced pressure to give diglycerol tetranitrate as a pale yellow oil in 97.3% yield.

The foregoing detailed description of the invention has been provided for the purpose of explaining the principles of the invention and its practical application, thereby enabling others skilled in the art to understand the invention for various embodiments and with various modifications as are suited to the particular use contemplated. This description is not intended to be exhaustive or to limit the invention to the precise embodiments disclosed. Modifications and equivalents will be apparent to practitioners skilled in this art and are encompassed within the spirit and scope of the appended claims.

## Claims

1. A method of synthesizing diglycerol tetranitrate, comprising:
forming a nitrating medium comprising a mixed acid phase and an inert organic phase, the mixed acid phase comprising, as ingredients, at least one nitronium ion source and at least one acid having sufficient strength to generate nitronium ions from the nitronium ion source, the inert organic phase comprising at least one organic liquid in which diglycerol is insoluble yet in which diglycerol tetranitrate is soluble;
combining the nitrating medium with diglycerol and nitrating the diglycerol in the mixed acid phase to form diglycerol tetranitrate, which is received into the inert organic phase;
separating the inert organic phase having the diglycerol tetranitrate dissolved therein from the mixed acid phase; and
recovering the diglycerol tetranitrate from the inert organic phase.

2. The method of claim 1, further comprising neutralizing any of the acid or nitronium ion source present in the inert organic phase subsequent to said separating of the inert organic phase from the mixed acid phase.

3. The method of claim 1, wherein the nitronium ion source comprises nitric acid and further wherein the acid comprises sulfuric acid.

4. The method of claim 3, wherein a molar ratio of the nitronium ion source to the diglycerol is at least 4:1 and not greater than about 8:1.

5. The method of claim 3, wherein a weight ratio of the nitric and sulfuric acids to water in the mixed acid phase is from about 100:0 to about 80:20.

6. The method of claim 3, wherein the organic liquid comprises at least one organic chlorocarbon.

7. The method of claim 6, wherein the chlorocarbon is dichloromethane.

8. The method of claim 3, wherein a weight ratio of the inert organic phase to the diglycerol tetranitrate is preferably at least 1:1.

9. The method of claim 3, wherein said nitrating of the diglycerol further comprises maintaining the nitrating medium in a range of from about 5°C to about 20°C.

10. The method of claim 3, further comprising neutralizing any of the acid or nitronium ion source present in the inert organic phase subsequent to said separating of the inert organic phase from the mixed acid phase.

11. A rocket motor assembly comprising a rocket motor case, a solid propellant grain loaded in the rocket motor case, and a nozzle in operative association with the rocket motor case to receive and discharge combustion products generated upon ignition of the solid propellant grain, the solid propellant grain comprising diglycerol tetranitrate synthesized according to the method of claim 1.
